Europäisches Patentamt

European Patent Office

Office européen des brevets

(11) Publication number: **0 272 057**
**A2**

(12) **EUROPEAN PATENT APPLICATION**

(21) Application number: 87310881.5

(51) Int. Cl.⁴: **A61K 31/47**

(22) Date of filing: 10.12.87

(30) Priority: 19.12.86 GB 8630419

(43) Date of publication of application:
22.06.88 Bulletin 88/25

(84) Designated Contracting States:
DE ES GB NL SE

(71) Applicant: FISONS plc
Fison House Princes Street
Ipswich Suffolk IP1 1QH(GB)

(72) Inventor: Wright, Paul
7 Thornhill Close
Bramcote Notingham, NG9 3FS(GB)

(74) Representative: Wright, Robert Gordon McRae
Fisons plc 12 Derby Road
Loughborough, Leicestershire LE11 0BB(GB)

(54) **Pharmaceutical compositions containing pyranoquinolines.**

(57) There are described pharmaceutical compositions containing as active ingredient 9-ethyl-6,9-dihydro-4,6-dioxo-10-propyl-4H-pyrano[3,2-g]-quinoline-2,8-dicarboxylic acid or a pharmaceutically acceptable salt thereof and a pharmaceutically acceptable adjuvant or excipient in a form suitable for rectal or oesophageal administration.

There is also described a method of treatment of certain gastrointestinal conditions, eg nutritional diseases and malabsorbtion syndrome, which comprises administration of the active ingredient to a patient suffering from such a condition.

EP 0 272 057 A2

# PHARMACEUTICAL COMPOSITIONS CONTAINING PYRANOQUINOLINES

This invention relates to pharmaceutical formulations, methods for their preparation and methods of treatment using them.

UK Patent 2022078 discloses a number of pyranoquinolines which are indicated for use in the treatment of, inter alia, reversible airways obstruction. These compounds are described as being administrered oesophageally or by inhalation. In general, however, the compounds are highly polar, hydrophilic molecules and as such would not be expected to be absorbed through the gut to a sufficient extent to provide therapeutic levels of the compounds in the sub-epithelial tissues.

UK Patent application 2157291 discloses a pressurised aerosol formulation of one of these pyranoquinolines, known as nedocromil sodium, which is the disodium salt of 9-ethyl-6,9-dihydro-4,6-dioxo-10-propyl-4H-pyrano[3,2-g]quinoline-2,8-dicarboxylic acid, for the treatment of reversible airways obstruction.

Surprisingly we have now found that compositions containing as active ingredient, 9-ethyl-6,9-dihydro-4,6-dioxo-10-propyl-4H-pyrano[3,2-g]-quinoline-2,8-dicarboxylic acid or a pharmaceutically acceptable salt thereof are useful in the treatment of certain conditions of the gastrointestinal tract. In addition, we have found that the compositions are also useful in treating certain food-related diseases, such as migraine, which have a symptomatic manifestation remote from the gastrointestinal tract. Surprisingly, we have also found that the compositions are useful in reducing the permeability of the gastrointestinal tract, which may lead to utility in the treatment of certain nutritional diseases.

According to the invention there is provided a pharmaceutical composition comprising 9-ethyl-6,9-dihydro4,6-dioxo-10-propyl-4H-pyrano[3,2-g]-quinoline-2,8-dicarboxylic acid or a pharmaceutically acceptable salt thereof and a pharmaceutically acceptable adjuvant or excipient in a form suitable for rectal or oesophageal administration, but not suitable for administration by inhalation.

We prefer the pharmaceutically acceptable salt to be an alkali metal salt. We particularly prefer the disodium salt, which is known as nedocromil sodium.

In order to produce suitable compositions the drug is worked up with inorganic or organic pharmaceutically acceptable adjuvants or excipients. Examples of such adjuvants are:

For tablets and dragees: Binders for example, cellulosic materials such as micro crystalline cellulose and methyl cellulose; disintegrating agents, for example starches, particularly maize starches; stabilisers, eg against hydrolysis of the active ingredients; flavouring agents, eg sugars; fillers; stearates and inorganic diluents, eg talc.

A particular tablet which may be mentioned is an effervescent tablet which disintegrates in a aqueous environment. Such tablets are particularly useful when the active ingredient is in the form of a salt, eg sodium, which is converted to the free acid in the highly acidic environment of the stomach.

The tablet preferably comprises from 10 to 80% by weight of a mixture of an alkali-metal or alkaline earth metal carbonate or bicarbonate and citric acid. The carbonate or bicarbonate may, for example, be sodium or potassium carbonate or bicarbonate, sodium bicarbonate being especially preferred. The tablet preferably comprises from about 10 to 40% by weight of the carbonate or bicarbonate and from about 5 to 35% by weight of the citric acid.

The tablet preferably contains from about 10 to 90% by weight of active ingredient, more preferably from about 40 to 80% and particularly from about 50 to 70%.

The carbonate or bicarbonate and acid are preferably present in stoichiometric amounts. Thus, the weight ratio of carbonate or bicarbonate to citric acid is preferably from about 1.2:1 to 1.8:1.

For syrups, suspensions or dispersions: A liquid vehicle in which the active ingredients may be dissolved or suspended, eg water; and suspending agents, eg cellulose derivatives, gums, etc.

For hard or soft capsules; Diluents, eg lactose; glidants, eg stearates; inorganic materials, eg silica or talc; stabilisers and dispersing agents.

For suppositories: Natural or hardened oils, waxes, etc. A large number of proprietary emulsifying bases are available and are suitable for use in suppositories, eg hydrogenated triglycerides of lauric acid with added monoglycerides, known under the trademark 'Witepsol' and gryceryl esters of lauric acid with a very small amount of glyceryl monostearate, known under the trademark 'Massupol'.

The compositions may also contain further adjuvants, for example a composition for use in tablets may contain lubricants and glidants to assist in tabletting, eg magnesium stearate or wetting agents to assist in granulation, eg dioctyl sodium sulphosuccinate.

The compositions of the present invention are useful in the treatment of certain disorders of the gastrointestinal tract. Conditions which may be mentioned include nutritional deficiencies, eg sprue, steatorrhea and malabsorption syndromes, particularly coeliac disease; regional entiritis, eg Crohn's disease and regional ileitis; distal colitis; proctitis; peptic ulceration; irritable bowel syndrome; and gastro-enteritis.

The compositions are also useful in the treatment of food-related diseases, which have a symptomatic manifestation remote from the gastro-intestinal tract, eg migraine, rhinitis, eczema, reversible obstructive airways disease, bronciectasis, aspergillosis, asbestosis, bird fancier's disease and farmer's lung.

The dosage to be administered will depend, amongst other things, on the particular condition to be treated and its severity. However, in general a total daily dosage of from about 25 to 100 mg of active ingredient, more preferably 100 to 400 mg, administered in smaller doses 2 to 4 times a day is found to be satisfactory. A dosage unit may conveniently contain from about 25 to 1000 mg of active ingredient. Administration preferably takes place a short time, eg 30 minutes, before the patient takes food.

The invention is illustrated, but in no way limited, by the following examples.

Examples

Effervescent TabletNedocromil Sodium      64.6 % w/w

| | |
|---|---|
| Sodium Bicarbonate BP | 18.6 % w/w |
| Citric Acid (anhydrous) BP | 14.2 % w/w |
| Magnesium Stearate BP | 2.6 % w/w |

Nedocromil sodium (323g, dry weight), anhydrous citric acid (71g) and sodium bicarbonate (93g) were thoroughly mixed and then granulated with isopropyl alcohol. The wet mass was partially dried on trays in a tray drying oven at 60°C for 30 minutes and the partially dried granules passed through a 10 mesh screen and then returned to the oven for a further 3 hours at 60°C. The dried granules were passed through an oscillating granulator fitted with a 20 mesh screen. The sieved granules were weighed and the required quantity of magnesium stearate calculated.

Magnesium stearate was sieved through a 40 mesh screen. The required quantity was weighed out and blended with the dried granules by shaking for 2 minutes. The granules were compressed into tablets containing 50mg nedocromil sodium using a single punch tabletting machine fitted with 5mm diameter normal concave punches. The tablets were stored in double sealed polythene bags containing silica gel sachets as desiccant.

Example 2

Non-effervescent TabletNedocromil Sodium      34% w/w

| | |
|---|---|
| Dicalcium phosphate | 34% w/w |
| Microcrystalline cellulose | 17% w/w |
| sodium carboxymethylcellulose | 9% w/w |
| Polyvinylpyrrolidone | 5% w/w |
| Magnesium stearate | 1% w/w |

The components were granulated by a method analogous to that described in Example 1, and the resulting granules compressed into tablets each containing 100mg nedocromil sodium.

Example 3

SuppositoriesNedocromil Sodium      10g
Theobroma Oil BP to      100g

Warmed nedocromil sodium was added to one half of the melted base and worked quickly into a smooth cream. This was then stirred into the remainder of the vase to form a homogenous mixture. The mixture was alowed to cool with stirring until the mixture was just pourable and then poured into suitably lubricated suppository moulds, which were cooled and trimmed with a sharp knife to give the suppositories.

Claims

1. A pharmaceutical composition comprising 9-ethyl-6,9-dihydro-4,6-dioxo-10-propyl-4H-pyrano-[3,2-g]quinoline-2,8-dicarboxylic acid or a pharmaceutically acceptable salt thereof and a pharmaceutical acceptable adjuvant or excipient in the form suitable for rectal or oesophageal administration, but not suitable for administration by inhalation.

2. A pharmaceutical composition according to Claim 1, wherein the pharmaceutically acceptable salt is the sodium salt.

3. A pharmaceutical composition comprising as active ingredient, 9-ethyl-6,9-dihydro-4,6-dioxo-10-propyl-4H-pyrano[3,2-g]quinoline-2,8-dicarboxylic acid or a pharmaceutically acceptable salt thereof or excipient, in a form suitable for rectal or oesophageal administration comprising unit dosages from 25 to 100mg of the active ingredient.

4. A composition according to any one of the preceding claims in solid form.

5. A composition according to Claim 4, in the form of a tablet or dragee.

6. A composition according to Claim 4, in the form of a hard or soft capsule.

7. A composition according to Claim 4, in the form of a suppository

8. A composition according to any one of Claims 1 to 3 in the form of a syrup.

9. A composition according to any one of Claims 1 to 3 in the form of a suspension or dispersion.

10. Use of 9-ethyl-6,9-dihydro-4,6-dioxo-10-propyl-4H-pyrano[3,2-g]-quinoline-2,8-dicarboxylic acid or a pharmaceutically acceptable salt thereof in the manufacture of a medicament for the treatment of a condition of the gastro-intestinal tract selected from the group comprising nutritional deficiencies, regional enteritis, distal colitis, proctitis, peptic ulceration, irritable bowel syndrome and gastro-enteritis.

11. Use of 9-ethyl-6,9-dihydro-4,6-dioxo-10-propyl-4H-pyrano[3,2-g]-quinoline-2,8-dicarboxylic acid or a pharmaceutically acceptable salt thereof in the manufacture of a medicament of per os treatment of a food related disease selected from the group comprising migraine, rhinitis, eczema, reversible obstructive airways disease, bronciestatis, aspergillosis, bird fancier's disease and farmer's lung.

Claims for the following Contracting State : ES

1. A method of preparing a pharmaceutical composition containing 9-ethyl-6,9-dihydro-4,6-dioxo-10-propyl-4H-pyrano[3,2-g]quinoline-2,8-dicarboxylic acid or a pharmaceutically acceptable salt thereof and a pharmaceutical acceptable adjuvant or excipient in a form suitable for rectal or oesophageal administration, but not suitable for administration by inhalation, which comprises mixing the ingredients.

2. A method according to Claim 1, wherein the pharmaceutically acceptable salt is the sodium salt.

3. A method of preparing a pharmaceutical composition containing as active ingredient 9-ethyl-6,9-dihydro-4,6-dioxo-10-propyl-4H-pyrano[3,2-g]-quinoline-2,8-dicarboxylic acid or a pharmaceutically acceptable salt thereof or excipient, in a form suitable for rectal or oesophageal administration comprising unit dosages from 25 to 1000mg of the active ingredient, which comprises mixing the ingredients.

4. A method according to any one of the preceding claims, wherein the composition is in solid form.

5. A method according to Claim 4, wherein the composition is in the form of a tablet or dragee.

6. A method according to Claim 4, wherein the composition is in the form of a hard or soft capsule.

7. A method according to Claim 4, wherein the composition is in the form of a suppository.

8. A method according to any one of Claims 1 to 3, wherein the composition is in the form of a syrup.

9. A method according to any one of Claims 1 to 3, wherein the composition is in the form of a suspension or dispersion.